# EUROPEAN PATENT SPECIFICATION

(11) **EP 3 202 449 B1**
(45) Date of publication and mention of the grant of the patent: **25.07.2018**
(21) Application number: 16305112.1
(22) Date of filing: 02.02.2016
(51) Int. Cl.: A61M 16/06, A61M 16/08, A61M 16/20

(54) **ELBOW CONNECTOR ASSEMBLY WITH ANTI-ASPHYXIA VALVE STRUCTURE FOR RESPIRATORY MASK**
WINKELVERBINDER MIT ANTI-ASPHYXIE-VENTILANORDNUNG FÜR ATEMMASKE
ENSEMBLE CONNECTEUR COUDÉ AVEC STRUCTURE DE VALVE ANTI-ASPHYXIE POUR MASQUE RESPIRATOIRE

(43) Date of publication of application: 09.08.2017
(73) Proprietor: Air Liquide Medical Systems, 92160 Antony (FR)
(72) Inventor: ALBERICI, Luca, 25030 RONCADELLE (BS) (IT); MASSERDOTTI, Fulvio, 25075 BRESCIA (IT); SANDONI, Giuseppe, 25124 BRESCIA (IT)
(74) Representative: Pittis, Olivier

(56) References cited:
- EP-A- 1 937 345
- EP-A1- 2 281 597
- WO-A1-02/051486
- WO-A1-2012/040791

## Description

The present invention concerns an elbow-shape connector comprising an improved anti-asphyxia valve for venting the CO₂-enriched gas expired by the patient to the atmosphere and a respiratory mask, especially a facial or full-face mask, suitable for treating sleep apnea comprising such an elbow-shape connector.

The respiratory masks that are used for treating sleep apnea are called "sleep apnea masks". A sleep apnea mask can be either a nasal or full face mask, comprising one or several exhalation orifices, also called venting or exhalation holes, ensuring the washout of the enriched-CO₂ gas expired by the patient. Venting holes are arranged either in the mask body as disclosed by EP-A-1582230, or in the tubular connector, typically an elbow-connector, used for fluidly connecting a flexible hose, or a similar fluid conduit, to the mask body as described by EP-A-2281597. Similar mask arrangements are also taught by WO-A-2005/051468 and EP-A-462701. Other mask arrangements with venting holes are also known from the documents WO 2012/040791 A1 and WO 02/051486 A1. However, common elbow-connectors with venting holes are not useable with facial or full-face masks, e.g. sleep apnea masks covering the nose and the mouth of the user. Indeed, for safety reasons, e.g. in case of default of the ventilator providing the respiratory gas under pressure, it is mandatory to use in combination with the facial mask, a connector including an anti-asphyxia valve allowing the patient to breath air through an opening arranged in the elbow-connector in case there is an inadequate pressure provided by the gas source delivering gas to the facial mask through the tubular connector.

Such a facial mask comprising a tubular connector with an anti-asphyxia valve assembly is disclosed by EP-A-1937345. The anti-asphyxia valve assembly comprises a flexible flap element arranged in the tubular connector for controlling the flow of gas circulating through the air opening located in the elbow portion of the connector.

However, such a connector with an anti-asphyxia valve assembly is not ideal because the connector is generally cumbersome and too heavy for ensuring a maximum comfort for the patient as well as a good stability of the mask, while used by the patient.

In other words, known sleep apnea facial masks comprising an anti-asphyxia valve assembly are not totally satisfying and need to be improved.

Hence, the goal of the present invention is to provide an improved elbow-connector suitable for respiratory masks, especially facial masks used for treating sleep apnea or other respiratory diseases.

The solution of the present invention is an elbow connector assembly comprising :
- a hollow elbow-shape structure comprising an inner gas path, and
- an anti-asphyxia valve structure comprising a flexible flap arranged in the inner gas path of the elbow-shape structure,
characterized in that the flexible flap is traversed by several holes and comprises a hemispherical-shape portion. The connector assembly according to the present invention can further comprise one or more of the following additional features:
- the inner gas path comprises an inlet orifice and an outlet orifice, and a port arranged between said inlet and outlet orifices, said port putting the inner gas path in fluid communication with the atmosphere, i.e. with ambient air.
- the flexible flap is arranged in the inner gas path of the elbow-shape structure between the inlet orifice and the port.
- the flexible flap is made of silicone.
- the hemispherical-shape portion comprises a peripheral annular border.
- the holes traversing the flexible flap have a conical shape.
- the anti-asphyxia valve structure comprises a fixation frame, the flexible flap being attached to said fixation frame.
- the fixation frame and the flexible flap are molded in one piece.
- the fixation frame of the anti-asphyxia valve structure is fixed to the inner wall of the hollow elbow-shape structure.
- the anti-asphyxia valve structure is arranged in the inner gas path of the elbow-shape structure so that the flexible flap can block off the port when pressurized gas circulates into the inner gas path from the inlet to the outlet orifice.
- the elbow-shape structure is made of plastic material.
- the elbow-shape structure is tubular, preferably it has a circular shaped cross-section.

The invention also concerns a respiratory mask comprising an elbow connector assembly according to the invention, preferably a facial mask, typically used for treating sleep apnea or other respiratory diseases.

The respiratory mask comprises a shell and a cushion, the connector assembly according to the invention being fluidly connected to the shell. Preferably, the connector assembly is rotatable when fixed to the shell of the respiratory mask.

Further, the cushion comprises a central aperture for receiving the patient's nose and mouth. It is made of soft, resilient, elastomeric material that comes into contact with the patient's face.

The respiratory mask further comprises a headgear comprising straps for fixing and maintaining the mask on the face of the user, i.e. the patient. Preferably the straps are made of fabric, polymer material or similar materials.

The shell forms a rigid or semi-rigid hollow mask body defining an internal breathing chamber or volume, wherein respiratory gas, such as air under pressure, is introduced via an inlet port arranged at the center of the mask body, which is fluidly connected to a gas feeding line, such as a flexible gas conduit, by means of the elbow connector assembly according to the present invention, thereby allowing air to be introduced in the breathing chamber that receives the nose and mouth of the patient. The mask body is preferably made of a polymer material, such as polycarbonate (PC), polypropylene (PP), ABS, nylon or polystyrene (PS).

In other words, in use, the patient introduces his/her nose and mouth into the internal volume of the breathing chamber of the mask body, i.e. the shell, and breathes the gas contained therein. The mask body preferably has a general triangular or quasi-triangular three-dimensional shape.

Preferably, the shell further comprises an expansion arm arranged on the top of the shell and projecting upwardly from said shell and comprising, at its free end, a forehead support with forehead cushion(s).

Furthermore, the invention also concerns a respiratory gas delivering assembly comprising :
- a gas source, preferably a CPAP or BPAP device, for delivering a pressurized respiratory gas, such as air under pressure (i.e. air pressure > 1 atm),
- a respiratory mask according to the present invention comprising an elbow connector assembly according to the present invention, preferably a facial mask that covers the nose and the mouth of the patient, and
- a flexible gas line, such as a plastic hose or the like, for conveying the gas delivered by the gas source to the elbow connector assembly of the respiratory mask thereby delivering said gas to the airways of the patient thanks to the mask.

Other features, aspects and advantages of the present invention will become apparent from the following detailed description when taken in conjunction with the accompanying drawings which illustrates, by way of examples, the present invention, among which :
- Figure 1 represents an elbow connector assembly according to the prior art,
- Figure 2 represents an embodiment of an elbow connector assembly according to the present invention,
- Figures 3 and 4 represent the structure of the anti-asphyxia valve structure of the elbow connector assembly of Figure 2, and
- Figure 5 represents a facial mask comprising the connector assembly of Figure 2.

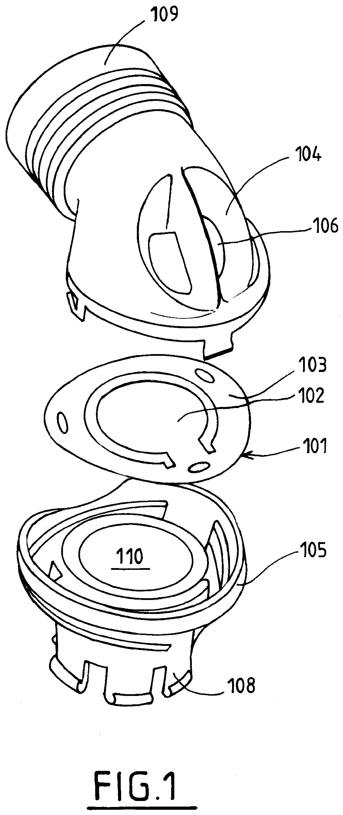

Figure 1 represents an embodiment of an elbow connector assembly 100 for a respiratory mask according to the prior art. It comprises a hollow elbow-shape structure, namely a tubular bent structure, preferably made of plastic material, made of two sub-units 104, 105 that are fixed together so as to define an inner gas path 110. It further comprises an anti-asphyxia valve structure comprising a valve element 101 and a large port 106 or opening. The valve element 101 comprises a fixation border 103 and a flexible flap 102 made of resilient material. The valve element 101 is "sandwiched", i.e. "squeezed", between the two sub-units 104, 105 so that the flaps 102 blocks off the gas path 110 when no pressurized gas circulated into said gas path 110. The fixation border 103 is used for fixing the valve element 101 between the two sub-units 104, 105 that are connected together, for example plugged one with the other.

In the present embodiment, the large port 106 is divided in two parallel passages by a separation wall 107; however, in other possible embodiments, the large port 106 can be divided in more than two passages, can comprise a grid or the like, or can, in opposite, not be divided, i.e. form a single undivided passage for gases. The large port 106 is located in the elbow portion of the elbow connector assembly 100.

When pressurized gas (i.e. at a pressure > 1 atm) circulates into the gas path 110, the flap 102 is bent by the gas pressure/flowrate so as to cover, such as a cap, the large port 106, thereby blocking the large port 106 off so that no air can enter into the gas path 110 through said large port 106. However, when no pressurized gas circulated into said gas path 110, e.g. due to a failure of the gas source, the flap 102 does not block off the large port 106 and air can enter into the inner path 110 and be inhaled by the patient.

This arrangement constitutes a safety valve system.

The elbow connector assembly 100 of Figure 1 further comprises connecting portions 108, 109 at its two ends 100a, 100b for fluidly connecting it, in a known manner, to a gas source, via a flexible hose or similar gas conduit, on the one hand, and to the body of a respiratory mask, on the other hand.

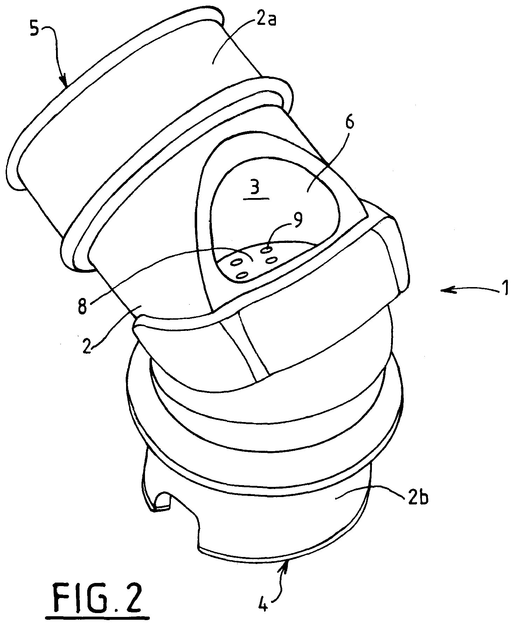

Figure 2 represents an embodiment of an elbow connector assembly 1 according to the present invention for a respiratory mask 20, especially a full-face or facial mask 20, such as a sleep apnea facial mask.

Similarly to the one of Figure 1, it comprises a hollow elbow-shape structure 2, namely a tubular bent structure, preferably made of plastic material, such as polycarbonate or similar, comprising an inner gas path 3 or gas passage and an anti-asphyxia valve structure 7 comprising a flexible flap 8 that is arranged in the inner gas path 3 of the elbow-shape structure 2, i.e. in the gas flow circulating in the inner gas path 3.

The flexible flap 8 is made of a flexible material, preferably silicone or the like. Said flexible flap 8 is further traversed by several holes 9, typically between 2 and 50 holes 9 through which expired gases can pass and be vented to the atmosphere, during the exhalation phases of the patient, as explained below. Those holes 9 are generally called exhalation holes. Preferably, each hole 9 has a size, i.e. diameter or the like, of less than 2 mm, preferably less than 1.5 mm, in particular between 0.5 and 1 mm.

The inner gas path 3 comprises an inlet orifice 4 and an outlet orifice 5. Pressurized gas normally circulates in the inner gas path 3 from the inlet orifice 4 to the outlet orifice 5 so as to be delivered afterwards to the respiratory mask 20, especially a facial mask, and then to the patient's airways.

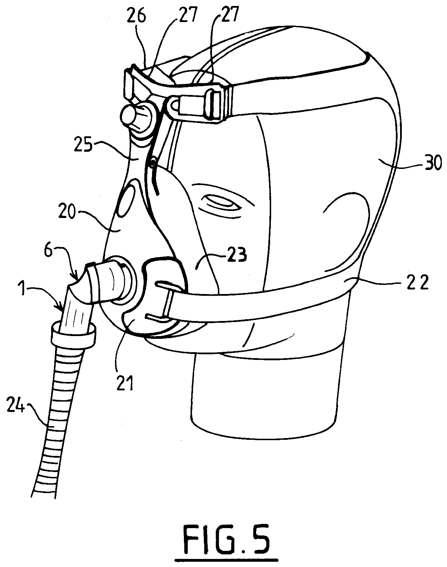

The shell 21 preferably has a general triangular or quasi-triangular three-dimensional shape as shown in Figure 5, for example. It further comprises an expansion arm 25 arranged on the top of the shell 21 and projecting upwardly from said shell 21, i.e. projecting away from the external surface of the shell 21. The expansion arm 25 comprises, at its free end, a forehead support 26 with one or several forehead cushions 27 that come into contact with the forehead of the patient 30.

The first end 2a of the elbow connector assembly 1 is configured for being connected, in a known manner, to the shell 21 of a respiratory mask 20, namely a facial mask sized for covering the nose and the mouth of a user, i.e. a patient 30, as shown in Figure 5. The mask 20 is held in position on the patient's face by means of a headgear 22 comprising straps made of fabric, polymer or similar. The mask 20 further comprises a cushion 23 fixed to the shell 21 and that contacts the patient's face, when the patient 30 wears the mask 20.

Likewise, the second end 2b of the elbow connector assembly 1 is configured for being connected, in a known manner, to a flexible gas line 24, namely a plastic hose or similar fluid conduit, receiving pressurized gas delivered by a gas source, such as a CPAP or BPAP device or any other suitable gas delivery apparatus.

Further, the elbow connector assembly 1 comprises a large port 6, i.e. an aperture, arranged between said inlet and outlet orifices 4, 5. The large port 6 is located in the elbow portion of the elbow connector assembly 1. The lumen of the inner gas path 3 is in fluid communication with the atmosphere through said large port 6, when said large port 6 is not blocked off by the flexible flap 8 (so called "rest" position), so as to constitute a safety passage for breathing ambient air, in case of failure or similar of the gas source, i.e. when no pressurized gas is fed to the connector assembly 1.

Indeed, the flexible flap 8 is mobile between at least two positions comprising:
- a "rest" position, wherein the flexible flap 8 block off the inner gas path 3, when no gas circulates into the inner gas path 3 (e.g. in case of failure of the gas source), and
- an "active" position, wherein the flexible flap 8 is bent and/or deformed by the gas pressure so as to cover the large port 6 thereby blocking off the large port 6.

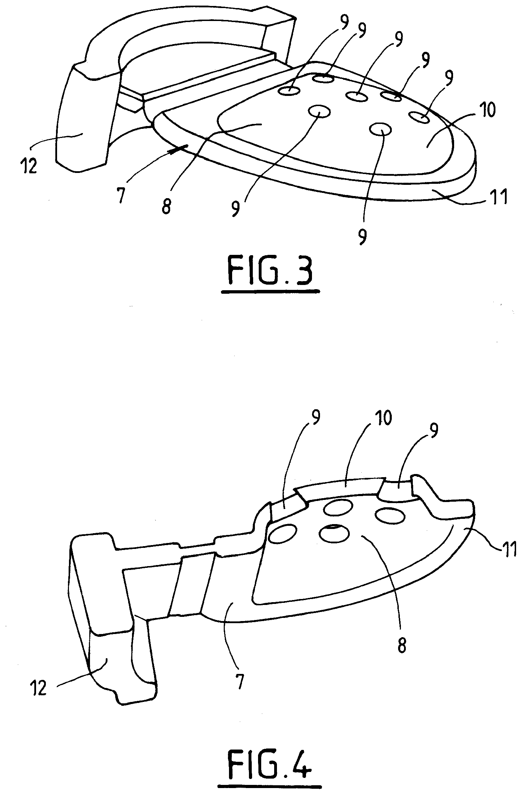

The anti-asphyxia valve structure 7 comprising the flexible flap 8 is detailed on Figures 3 and 4.

The flexible flap 8 comprises a hemispherical-shape portion 10 that is located at the center of the anti-asphyxia valve structure 7.

The hemispherical-shape portion 10 comprises the exhalation holes 9. Preferably, the exhalation holes 9 have a conical shape (i.e. tronconical shape) with their larger diameter in fluid communication with the lumen of the inner path 3 and their smaller diameter in fluid communication with the atmosphere thereby limiting the noise level due to expired gases, as shown in Figure 4.

Further, the hemispherical-shape portion 10 also comprises a peripheral annular border 11, i.e. a border forming a crown around the hemispherical-shape portion 10, and a fixation frame 12. The flexible flap 8 is attached to the fixation frame 12. Preferably, the fixation frame 12 and the flexible flap 8 are molded in one piece, for instance made of silicone or the like. The anti-asphyxia valve structure 7 is fixed to the inner wall of the hollow elbow-shape structure 2 by means of the fixation frame 12 for maintaining the anti-asphyxia valve structure 7 in position into the inner path 3.

Of course, the anti-asphyxia valve structure 7 can have a different architecture. For instance, the central region of the flexible flap 8 can have a flat top. According to the present invention, the exhalation holes 9 are directly arranged through the flap 8, preferably in its central portion having a hemispherical shape 10, thereby venting to the atmosphere at least a part of the gases expired by the patient during the expiration phases of the breathing or respiratory cycle of said patient.

This allows having a more compact and lighter elbow thereby ensuring a maximum comfort and stability of the full face mask on the patient's face.

The mask according to the present invention is useable for treating respiratory failures or disorders, such as sleep apnea or the like.

## Claims

1. Elbow connector assembly (1) comprising :
- a hollow elbow-shape structure (2) comprising an inner gas path (3), and
- an anti-asphyxia valve structure (7) comprising a flexible flap (8) arranged in the inner gas path (3) of the elbow-shape structure (2),
**characterized in that** the flexible flap (8) is traversed by several holes (9) and comprises a hemispherical-shape portion (10).

2. Elbow connector assembly according to the previous Claim, **characterized in that** the inner gas path (3) comprises an inlet orifice (4) and an outlet orifice (5), and a port (6) arranged between said inlet and outlet orifices (4, 5), said port (6) putting the inner gas path (3) in fluid communication with the atmosphere.

3. Elbow connector assembly according to Claim 2, **characterized in that** the flexible flap (8) is arranged in the inner gas path (3) of the elbow-shape structure (2) between the inlet orifice (4) and the port (6).

4. Elbow connector assembly according to any one of the previous Claims, **characterized in that** the flexible flap (8) is made of silicone.

5. Elbow connector assembly according to Claim 1, **characterized in that** the hemispherical-shape portion (10) comprises a peripheral annular border (11).

6. Elbow connector assembly according to any one of the previous Claims, **characterized in that** the holes (9) traversing the flexible flap (8) have a conical shape.

7. Elbow connector assembly according to any one of the previous Claims, **characterized in that** the anti-asphyxia valve structure (7) comprises a fixation frame (12), the flexible flap (8) being attached to said fixation frame (12).

8. Elbow connector assembly according to Claim 7, **characterized in that** the fixation frame (12) and the flexible flap (8) are molded in one piece.

9. Elbow connector assembly according to claim 7 or claim 8, **characterized in that** the fixation frame (12) of the anti-asphyxia valve structure (7) is fixed to the inner wall of the hollow elbow-shape structure (2).

10. Elbow connector assembly according to Claim 2, **characterized in that** the anti-asphyxia valve structure (7) is arranged in the inner gas path (3) of the elbow-shape structure (2) so that the flexible flap (8) can block off the port (6) when pressurized gas circulates through the inner gas path (3) from the inlet (4) to the outlet (5) orifice.

11. Respiratory mask (20) comprising an elbow connector assembly (1) according to any one of the previous claims, preferably a facial mask.

12. Respiratory mask (20) according to Claim 11, **characterized in that** it comprises a shell (21), the connector assembly (1) being fluidly connected to the shell (21).

13. Respiratory mask (20) according to Claims 11 or 12, **characterized in that** it further comprises a headgear (22) comprising straps.

14. Respiratory mask (20) according to Claim 12, **characterized in that** the shell (21) comprises a cushion (23).

15. Respiratory gas delivering assembly comprising :
- a gas source, preferably a CPAP or BPAP device, for delivering a pressurized respiratory gas,
- a respiratory mask (20) according to any one of Claims 11 to 14, preferably a facial mask, and
- a flexible gas line (24) for conveying the gas delivered by the gas source to the elbow connector assembly (1) of the respiratory mask (20).

## Patentansprüche

1. Winkelverbinderanordnung (1), umfassend:
- eine hohle ellbogenförmige Struktur (2), umfassend einen inneren Gasweg (3) und
- eine Anti-Asphyxie-Ventilanordnung (7), umfassend eine flexible Klappe (8), die im inneren Gasweg (3) der ellbogenförmigen Struktur (2) angeordnet ist,
**dadurch gekennzeichnet, dass** die flexible Klappe (8) von mehreren Löchern (9) durchquert wird und einen halbkugelförmigen Abschnitt (10) aufweist.

2. Winkelverbinderanordnung nach dem vorstehenden Anspruch, **dadurch gekennzeichnet, dass** der innere Gasweg (3) eine Einlassöffnung (4) und eine Auslassöffnung (5) und einen Anschluss (6), der zwischen der Einlass- und Auslassöffnung (4, 5) angeordnet ist, umfasst, wobei der Anschluss (6) den inneren Gasweg (3) mit der Atmosphäre in Fluidverbindung bringt.

3. Winkelverbinderanordnung nach Anspruch 2, **dadurch gekennzeichnet, dass** die flexible Klappe (8) im inneren Gasweg (3) der ellbogenförmigen Struktur (2) zwischen der Einlassöffnung (4) und dem Anschluss (6) angeordnet ist.

4. Winkelverbinderanordnung nach einem der vorstehenden Ansprüche, **dadurch gekennzeichnet, dass** die flexible Klappe (8) aus Silicon hergestellt ist.

5. Winkelverbinderanordnung nach Anspruch 1, **dadurch gekennzeichnet, dass** der halbkugelförmige Abschnitt (10) einen umlaufenden ringförmigen Rand (11) umfasst.

6. Winkelverbinderanordnung nach einem der vorstehenden Ansprüche, **dadurch gekennzeichnet, dass** die Löcher (9), welche die flexible Klappe (8) durchqueren, eine konische Form aufweisen.

7. Winkelverbinderanordnung nach einem der vorstehenden Ansprüche, **dadurch gekennzeichnet, dass** die Anti-Asphyxie-Ventilanordnung (7) einen Befestigungsrahmen (12) umfasst, wobei die flexible Klappe (8) an dem Befestigungsrahmen (12) angebracht ist.

8. Winkelverbinderanordnung nach Anspruch 7, **dadurch gekennzeichnet, dass** der Befestigungsrahmen (12) und die flexible Klappe (8) einstückig geformt sind.

9. Winkelverbinderanordnung nach Anspruch 7 oder Anspruch 8, **dadurch gekennzeichnet, dass** der Befestigungsrahmen (12) der Anti-Asphyxie-Ventilanordnung (7) an der Innenwand der hohlen ellbogenförmigen Struktur (2) befestigt ist.

10. Winkelverbinderanordnung nach Anspruch 2, **dadurch gekennzeichnet, dass** die Anti-Asphyxie-Ventilanordnung (7) im inneren Gasweg (3) der ellbogenförmigen Struktur (2) angeordnet ist, so dass die flexible Klappe (8) den Anschluss (6) versperren kann, wenn Druckgas durch den inneren Gasweg (3) von der Einlass- (4) zu der Auslassöffnung (5) zirkuliert.

11. Atemmaske (20) mit einem Winkelverbinder (1) nach einem der vorstehenden Ansprüche, vorzugsweise eine Gesichtsmaske.

12. Atemmaske (20) nach Anspruch 11, **dadurch gekennzeichnet, dass** sie eine Außenhaut (21) umfasst, wobei die Verbindungsanordnung (1) mit der Außenhaut (21) in Fluidverbindung steht.

13. Atemmaske (20) nach Anspruch 11 oder 12, **dadurch gekennzeichnet, dass** sie ferner eine Kopfhalterung (22) umfasst, die Riemen umfasst.

14. Atemmaske (20) nach Anspruch 12, **dadurch gekennzeichnet, dass** die Außenhaut (21) eine Polsterung (23) umfasst.

15. Atemgas-Abgabeanordnung, umfassend:
- eine Gasquelle, vorzugsweise eine CPAP- oder BPAP-Vorrichtung zum Abgeben eines druckbeaufschlagten Atemgases,
- eine Atemmaske (20) nach einem der Ansprüche 11 bis 14, vorzugsweise eine Gesichtsmaske, und
- eine flexible Gasleitung (24) zum Befördern des Gases, das der Winkelverbinderanordnung (1) der Atemmaske (20) von der Gasquelle zugeführt wird.

## Revendications

1. Ensemble connecteur coudé (1) comprenant :
- une structure de forme coudée creuse (2) comprenant un trajet de gaz interne (3) et
- une structure de vanne anti-asphyxie (7) comprenant un volet souple (8) agencée dans le trajet de gaz interne (3) de la structure de forme coudée (2),
**caractérisé en ce que** le volet souple (8) est traversé par plusieurs trous (9) et comprend une partie de forme hémisphérique (10).

2. Ensemble connecteur coudé selon la revendication précédente, **caractérisé en ce que** le trajet de gaz interne (3) comprend un orifice d'entrée (4) et un orifice de sortie (5) ainsi qu'une ouverture (6) agencée entre lesdits orifices d'entrée et de sortie (4, 5), ladite ouverture (6) mettant le trajet de gaz interne (3) en communication fluidique avec l'atmosphère.

3. Ensemble connecteur coudé selon la revendication 2, **caractérisé en ce que** le volet souple (8) est agencé dans le trajet de gaz interne (3) de la structure de forme coudée (2) entre l'orifice d'entrée (4) et l'ouverture (6).

4. Ensemble connecteur coudé selon l'une quelconque des revendications précédentes, **caractérisé en ce que** le volet souple (8) est constitué de silicone.

5. Ensemble connecteur coudé selon la revendication 1, **caractérisé en ce que** la partie de forme hémisphérique (10) comprend une bordure annulaire périphérique (11).

6. Ensemble connecteur coudé selon l'une quelconque des revendications précédentes, **caractérisé en ce que** les trous (9) traversant le volet souple (8) ont une forme conique.

7. Ensemble connecteur coudé selon l'une quelconque des revendications précédentes, **caractérisé en ce que** la structure de vanne anti-asphyxie (7) comprend un bâti de fixation (12), le volet souple (8) étant fixé audit bâti de fixation (12).

8. Ensemble connecteur coudé selon la revendication 7, **caractérisé en ce que** le bâti de fixation (12) et le volet souple (8) sont moulés d'un seul tenant.

9. Ensemble connecteur coudé selon la revendication 7 ou la revendication 8, **caractérisé en ce que** le bâti de fixation (12) de la structure de vanne anti-asphyxie (7) est fixé à la paroi interne de la structure de forme coudée creuse (2).

10. Ensemble connecteur coudé selon la revendication 2, **caractérisé en ce que** la structure de vanne anti-asphyxie (7) est agencée dans le trajet de gaz interne (3) de la structure de forme coudée (2) de sorte que le volet souple (8) puisse bloquer l'ouverture (6) lorsque du gaz sous pression circule à travers le trajet de gaz interne (3) de l'orifice d'entrée (4) à l'orifice de sortie (5).

11. Masque respiratoire (20) comprend un ensemble connecteur coudé (1) selon l'une quelconque des revendications précédentes, de préférence un masque facial.

12. Masque respiratoire (20) selon la revendication 11, **caractérisé en ce qu'**il comprend une coque (21), l'ensemble connecteur (1) étant en communication fluidique avec la coque (21).

13. Masque respiratoire (20) selon les revendications 11 ou 12, **caractérisé en ce qu'**il comprend en outre une structure de tête (22) comprenant des courroies.

14. Masque respiratoire (20) selon la revendication 12, caractérisé en ce la coque (21) comprend un amortisseur (23).

15. Ensemble de fourniture de gaz respiratoire, comprenant :
- une source de gaz, de préférence un dispositif CPAP ou BPAP, pour fournir un gaz respiratoire sous pression,
- un masque respiratoire (20) selon l'une quelconque des revendications 11 à 14, de préférence un masque facial, et
- une conduite de gaz souple (24) pour acheminer le gaz délivré par la source de gaz à l'ensemble connecteur coudé (1) du masque respiratoire (20).
